# EUROPÄISCHE PATENTSCHRIFT

(11) **EP 0 622 056 B1**
(45) Veröffentlichungstag und Bekanntmachung des Hinweises auf die Patenterteilung: **17.06.1998**
(21) Anmeldenummer: 94850065.7
(22) Anmeldetag: 26.04.1994
(51) Int. Cl.: A61F 2/00, A61F 2/30, A61C 8/00, A61B 17/58, A61F 2/42

(54) **Implantierbares Verankerungsorgan zur Aufnahme von Prothesen u.dgl.**
Implantable anchoring organ for receiving prostheses and the like
Organe d'ancrage implantable pour la réception de prothèses et d'objets similaires

(30) Priorität: 27.04.1993 SE 9301405
(43) Veröffentlichungstag der Anmeldung: 02.11.1994
(73) Patentinhaber: MEDEVELOP AKTIEBOLAG, S-402 29 Göteborg (SE)
(72) Erfinder: Branemark, Per-Ingvar, S-431 69 Mölndal (SE)
(74) Vertreter: Karlsson, Leif Karl Gunnar

(56) Entgegenhaltungen:
- WO-A-89/09579
- WO-A-91/18556
- DE-A- 2 628 443
- DE-U- 8 812 806

## Beschreibung

Die vorliegende Erfindung betrifft ein zur Implantation in Geweben vorgesehenes, hauptsächlich rotationssymmetrisch ausgebildetes Verankerungsorgan zur Halterung von Prothesen u.dgl., sowie eine derartige Verankerungsorgane umfassende Verankerungsvorrichtung, gemäss der Präambel von Anspruch 1 bzw. 18.

In erster Hand betrifft die Erfindung grundsätzlich ein rotationssymmetrisch ausgebildetes und aus einem gewebeverträglichen Material bestehendes Verankerungsorgan zum Anschluss von Prothesen, künstlichen Gelenkkomponenten u.dgl., wobei das Verankerungsorgan ein von seinem hinteren Ende ausgehendes und in Richtung zum entgegengesetzten Einführungsende verlaufendes Aussengewinde aufweist.

Solche schraubenförmige Verankerungsorgane sind bekannt und beispielsweise in US-A- 5 0643 425 beschrieben; sie werden auch seit langer Zeit mit grossem Erfolg, hauptsächlich im Dentalsektor, verwendet und unter anderem von NobelPharma AB under dem Warenzeichen Brånemark System® verkauft.

Hierbei bestehen die Verankerungsorgane in der Regel aus Titan oder weisen zumindest eine Titanbeschichtung auf und sind, zur Erzielung einer optimaler Osseointegration, mit einer Mikrodellen aufweisenden Oberfläche versehen. Eine solche Oberflächenstruktur ist beispielsweise in US-A- 4 330 891 beschrieben.

In den meisten Fällen und besonders im Zusammenhang mit zahntechnischen Applikationen werden diese schraubenförmigen Implantate axialen Belastungen unterworfen, indem sie an ihrem hinteren Ende, dem sogenannten Applikationsende, unter Zwischenschaltung geeigneter Distanzelemente künstliche Zähne oder Zahnbrücken tragen.

In letzter Zeit hat man derartige schraubenförmige Verankerungsorgane auch zur Verankerung von beispielsweise künstlichen Hüftgelenk-, Fussgelenk- und Handgelenkkonstruktionen verwendet - vgl. hierzu beispielsweise US-A- 5 041 139 und US-A- 5 108 444 -, wobei die Schrauben auch quer zur Längsrichtung des Knochens verankert wurden.

Aus WO-A-8909579 ist ferner ein zur Implantation in Geweben vorgesehenes, hauptsächlich rotationssymmetrisch ausgebildetes und aus einem gewebeverträgligen Material bestehendes Verankerungsorgan bekannt, welches an der Aussenfläche mit einem Schraubengewinde versehen werden kann. Die Mantelfläche des Verankerungsorgans ist mit einem, in Abstand vom Einführungsende angeordneten schlitzförmigen Spalt versehen. Dieser Spalt reicht nicht bis zu einem Ende des Verankerungsorgans, so dass ein Element nur in Radialrichtung des Organs eingeführt werden kann.

Aufgabe vorliegender Erfindung ist ein Verankerungsorgan der obengenannten Art zu schaffen, das teils zur Aufnahme von sowohl axialen als auch in Querrichtung auftretenden, von am Verankerungsorgan direkt oder über geeignete Zwischenstücke anschliessbaren Prothesen, künstlichen Gelenke u.dgl. ausgehenden Belastungen eingerichtet ist, teils in optimaler Weise in einem im Gewebe vorpräparierten Hohlraum lagefixierbar ist, und teils die Möglichkeit eines direkten Anschlusses aktueller Prothesenteile bietet. Hierdurch werden bedeutende operationstechnische Vorteilen erreicht und auch ein Minimum von früher notwendigen Anschlussteilen für verschiedene Verbindungsvorrichtungen benötigt, wodurch auch bedeutend weniger Platz in Anspruch genommen wird.

Diese Aufgabe wird bei einem gattungsgemässen Verankerungsorgan erfindungsgemäss durch die Merkmale des gekennzeichnenden Teils des Anspruchs 1 gelöst.

Durch diese prinzipielle Ausführung des Verankerungsorgans werden zum ersten Male Voraussetzungen zum Anbringen von Prothesenteilen geschaffen, die das Verankerungsorgan hauptsächlich senkrecht zu dessen Längsrichtung belasten, sodass man erstmalig die Festigkeit des Verankerungsorgans in Querrichtung optimal ausnutzen kann. Gleichzeitig damit werden bedeutende operationstechnische Vereinfachungen erzielt, da ein in dieser Weise ausgebildetes schraubenförmiges Verankerungsorgan grundsätzlich zur Belastung sowohl in axialer Richtung als auch zur Belastung in Richtung quer zur Längsrichtung der Schraube verwendet werden kann. Hierdurch kann das Verankerungsorgan sehr nahe an der aktuellen Gelenkverbindung angebracht werden kann und, was den Dentalbereich anbelangt, die Schraube von der Vorderseite her in den Gaumen eingesetzt werden kann, im Gegensatz zur jetzigen Technik, wo dieses Einsetzen von oben her erfolgt.

Nach einer bevorzugten Ausführungsform der Erfindung ist der genannte Spalt bei der Applikation des Verankerungsorgans im Gewebe und während dessen Osseointegration mit dem Gewebe der freipräparierten Aussparung zur lösbaren Aufnahme einer den genannten Spalt abschirmenden Leiste vorgesehen. Dadurch kann beispielsweise eine Halterung für Prothesen nach der Verankerung des Organs im umgebenden Gewebe leicht gegen die genannte Deckleiste ausgetauscht werden, indem das Organ nach Entfernung der Deckleiste in den Schlitz eingeführt wird.

Zweckmässig kann hierbei der genannte Spalt eine in das Verankerungsorgan gefräste Nut sein, deren Begrenzungskanten zur Fixierung der lösbaren Deckleiste vorgesehen sind.

Nach einer bevorzugten Ausführungsform der Erfindung erstreckt sich eine Innenbohrung vom hinteren Ende des Verankerungsorgans aus, wobei diese Bohrung sich in der Längsrichtung des Organs - d. h. in Richtung zum Einführungsende erstreckt - und mit dem spaltförmigen Schlitz in Verbindung steht und im wesentlichen mit der Länge des spaltförmigen Schlitzes übereinstimmt oder etwas länger als der Schlitz ist.

Zweckmässig besteht die Deckleiste aus einem zum lösbaren Einschieben in den spaltförmigen Schlitz mittels wenigstens einer axialen Auskragung und zum Abdecken des spaltförmigen Schlitzes bei wenigstens teilweiser Ausfüllung der Innenbohrung vorgesehenen Mitnehmer.

Zweckmässig ist der Mitnehmer hierbei an seiner aus dem oberen Ende des Organes und/oder aus der Spalte herausragenden Auskragung zum Anschluss an Zwischenstücke zum Halten der genannten Prothesenteile, künstlichen Gelenkkomponenten u.dgl. ausgebildet.

Durch diese bevorzugte Ausführungsform wird eine wesentlich vereinfachte Lagefixierung des schraubenförmigen Verankerungsorgans in dem im voraus freipräparierten Hohlraum im Knochengewebe erzielt, indem der Mitnehmer insgesamt zum Hineintreiben und Einschrauben des Verankerungsorgans während dessen Lagefixierung verwendet werden kann.

Bei dieser bevorzugten Ausführungsform kann der Mitnehmer aus der Innenbohrung herausgenommen werden, nachdem das Verankerungsorgan mit dem umgebenden Gewebe osseointegriert ist, und kann durch einen Halter für Prothesenteile ersetzt werden, der in etwa gleicher Weise wie der Mitnehmer ausgebildet ist und somit beim Einschieben in den Spalt sowohl diesen als auch einen Teil der Innernbohrung ausfüllt.

Nach einer weiteren Ausführungsform kann der schlitzförmige Spalt mit hauptsächlich von diesem rechtwinklig ausgehenden Seitenspalten zur Lagefixierung des Mitbringers oder zur Lagefixierung eines in die Spalte einsetzbaren Halters für Prothesenteile ausgebildet sein.

Zweckmässig kann der Mitnehmer nach dem Einführen des Verenkerungsorgans im Gewebe durch einen Halter von grundsätzlich gleichartiger Ausbildung ersetzt werden, wobei der Halter mit einem an der Grundplatte vorgesehenen Auskragung zum Befestigen von Prothesenteilen u.dgl. versehen ist.

Hierbei kann, wie in den Ansprüchen 10 - 16 angegeben, der Halter auf verschiedenste Weise lagefixiert werden.

Die Erfindung umfasst auch mit oben beschriebenen Verankerungsorganen versehene Verankerungsvorrichtungen, die vorteilhaft zur Rekonstruktion von Gelenken, wie Fuss-, Knie-, Hüft-, Armgelenken u.dgl., verwendet werden können.

Eine derartige Verankerungsvorrichtung, gemäss der Präambel von Anspruch 18, umfasst die Merkmale des kennzeichnenden Teils des Anspruchs 18.

Nach einer geeigneten Ausführungsform der Erfindung kann eine derartige Verankerungsvorrichtung zwei oder mehrere Verankerungsorgane umfassen und einen Implantatkörper mit zwei oder mehreren Kopplungsorganen aufweisen.

Weitere Ausführungsbeispiele der Erfindung gehen aus den in den abhängigen Ansprüchen angegebenen Merkmalen hervor.

Die Erfindung wird nachstehend an Hand einiger in den beigefügten Zeichnungen gezeigten Ausführungsbeispielen näher erläutert, es zeigen
- Fig. 1: eine perspektivische Darstellung eines Verankerungsorganes, welches nicht die Erfindung darstellt,
- Fig. 2: eine perspektivische Darstellung einer Ausführungsform eines erfindungsgemäss vorgeschlagenen Verankerungsorganes,
- Fig. 3-6: verschiedene Ausführungformen von erfindungsgemäss vorgeschlagenen Abdeckleisten in perspektivischer Darstellung mit dazugehörenden Schnitten durch das Verankerungsorgan,
- Fig. 7: eine perspektivische Darstellung einer dritten Ausführungsform eines erfindungsgemäss vorgeschlagenen Verankerungsorgans,
- Fig. 8: einen Mitnehmer in perspektivischer Darstellung,
- Fig. 9: einen Prothesenhalter in perspektivischer Darstellung,
- Fig. 10: eine perspektivische Darstellung mit aufgelösten Einzelteilen eines Verankerungsorganes nach Fig. 6 mit dazugehörendem Mitnehmer nach Fig. 7 samt einer Endverschlusskappe, letztere teilweise im Schnitt,
- Fig. 11: verschiedene Schnitte durch das in Fig. 6 gezeigte hintere Ende mit zwei verschiedenen Verriegelungen der genannten Kappe,
- Fig. 12: eine perspektivische Darstellung mit aufgelösten Einzelteilen eines erfindungsgemäss vorgeschlagenen Verankerungsorganes mit Halterung und Organ zur Lagefixierung,
- Fig. 13-14: eine Weiterentwicklung des Gegenstandes nach Fig.10 in einer Darstellung mit aufgelösten Einzelteilen bzw. im Schnitt,
- Fig. 15: einen Längsschnitt durch ein erfindungsgemäss vorgeschlagenes Verankerungsorgan mit eingesetztem Halter,
- Fig. 16-17: Schnitte durch den Gegenstand gemäss Fig. 14 entlang der Linie B - B, wobei Fig. 16 zeigt, wie der Halter durch Keilwirkung in seiner Lage fixiert werden kann,
- Fig. 18: drei verschiedene alternative Lösungen für eine Lagefixierung des Halters im Schlitz des Verankerungsorganes,
- Fig. 19: eine perspektivische Darstellung einer solchen, in Fig. 17 schematisch angedeuteten Lagefixierung des Halters,
- Fig. 20: einen Längsschnitt durch eine weitere Ausführungsform des Verankerungsorgans mit dazugehörendem Halter,
- Fig. 21: einen Schnitt entlang der Linie A - A in Fig. 19,
- Fig. 22: eine perspektivische Darstellung eines künstlichen Fussgelenks mit zwei erfindungsgemässen Verankerungvorrichtungen zur Verankerung im Talus bzw. in der Tibia, wobei nur eines von vier Verankerungsorganen gezeigt wird,
- Fig. 23: einen Vertikalschnitt durch das Zentrum des Gegenstandes in Fig. 1 und senkrecht zum angebrachten Verankerungsorgan, wobei alle vier Verankerungsschrauben gezeigt werden, unter anderem dadurch vereinfacht, dass die Einpassung der Taluskugel bzw. der Tibiaschale in den ensprechenden Implantatkörper weggelassen wurde,
- Fig. 24: die Tibiaschale des in Fig. 22 gezeigten Gegenstands, in derselben Ansicht,
- Fig. 25: den Tibiaimplantatkörper des in Fig. 22 gezeigten Gegenstands, in derselben Ansicht,
- Fig. 26: die Tibiaschale gemäss Fig. 24 im Schnitt (vergl. A - A, Fig. 30), senkrecht gegen eine der Seitenwände und die Rücken des Tibiaimplantatkörpers sowie der von den fluchtend angeordneten Gelenkachsen festgelegten Ebene,
- Fig. 27: die Taluskugel des in Fig. 22 gezeigten Gegenstands, in derselben Ansicht,
- Fig. 28: den Talusimplantatkörper des in Fig. 22 gezeigten Gegenstands, in perspektivischer Darstellung seiner an die Taluskugel anliegenden Seite,
- Fig. 29: das Verankerungsorgan des Gegenstands in Fig. 22, in perspektivischer Darstellung, wobei der in Längsrichtung vorgesehene Schlitz gezeigt wird,
- Fig. 30: den mit einer Verschlusskappe versehenen Tibiaimplantatkörper gemäss Fig. 25,
- Fig. 31: die Verschlusskappe für den Tibiaimplantatkörper gemäss Fig. 30, im Schnitt A - A,
- Fig. 32: den mit einer Verschlusskappe versehenen Talusimplantatkörper nach Fig. 28 und
- Fig. 33: den Ersatz eines natürlichen Gelenks durch eine erfindungsgemäss verankerte Gelenkprothese.

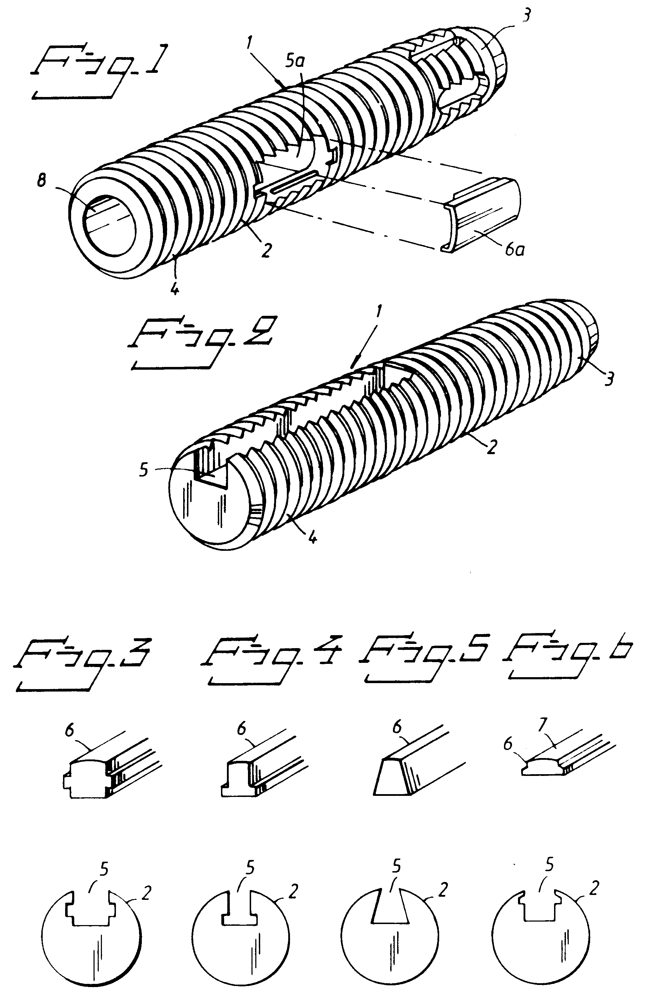

In der Zeichnung wird in Fig. 1 ein Verankerungsorgan veranschaulicht, das nicht die Erfindung darstellt.

Das Verankerungsorgan wird allgemein mit 1 bezeichnet und zeigt grundsätzlich den Aufbau eines rotationssymmetrischen Körpers in Form einer Schraube mit Aussengewinde 2, das sich in der gezeigten Ausführung zwischen den beiden Kurzenden erstreckt. Des eine Ende 3 der Schraube verjüngt sich etwas konisch und wird im folgenden als Einführungsende bezeichnet, d. h. ist das Ende, mittels welchem die Schraube anfangs in eine im voraus gefertigte Bohrung im Gewebe eingeschraubt werden soll, wobei die Bohrung einen etwas geringeren Durchmesser als der Aussendurchmesser der Schraube aufweist. Das entgegengesetzte Ende der Schraube wird mit 4 und im folgenden als hinteres Ende bezeichnet.

Das in Fig. 1 bezeigte Verankerungsorgan ist mit einer vom hinteren Ende ausgehenden Innerbohrung 8 und mit einer in ihrer Mantelfläche vorgesehenen schlitzförmigen Ausnehmung 5a versehen, die normalerweise von einer Abdeckleiste 6a abgedeckt wird, wobei letztere auf geeignete Weise lösbar in den die Ausnehmung 5a begrenzenden Kanten befestigt ist. Beim Einschrauben des Organes 1 in eine im Gewebe im voraus angebrachten Bohrung verschliesst die Abdeckleiste 6a somit die Ausnehmung 5a und kann - wie weiter unten näher beschrieben - nach Anschluss geeigneter Zwischenstücke zur Aufnahme von Prothesenteilen u.dgl. leicht entfernt werden. Die hier gezeigte schlitzförmige Ausnehmung 5a kann natürlicherweise auch in einem Winkel, vorzugsweise in rechtem Winkel, zur Längsachse des Verankerungsorgans vorgesehen sein. Durch eine solche Ausnehmung 5a, die zweckmässigerweise auf halber Länge des Organs liegt, kann man erstmals auch eine Querbelestung des Verankerungsorgans erreichen.

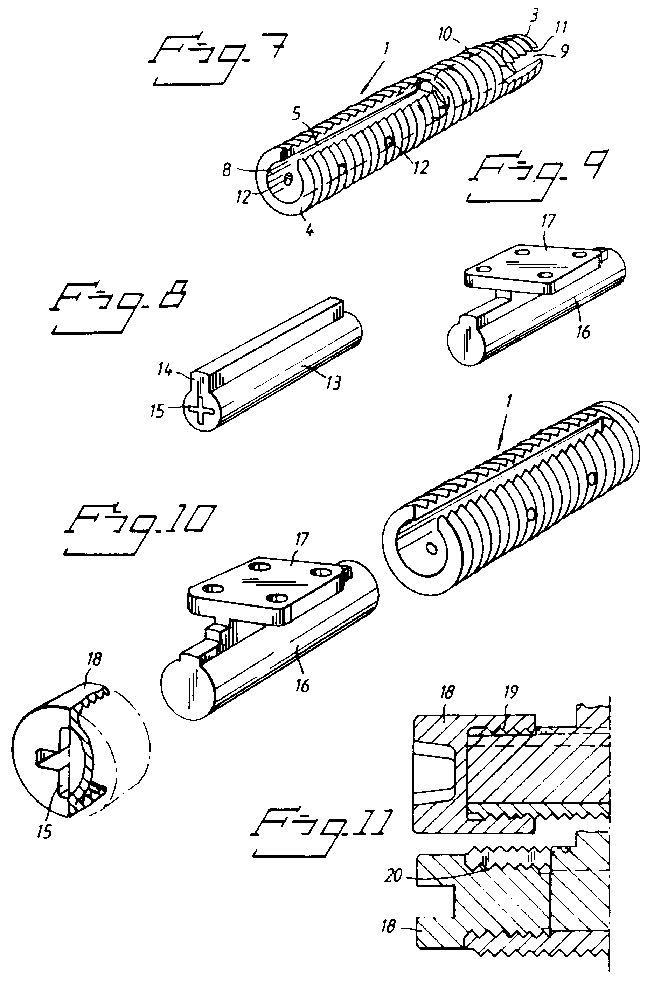

Anstelle einer schlitzförmigen Ausnehmung besitzt die in Fig. 2 gezeigte erfindungsgemässe Ausführungsform einen vom hinteren Ende der Schraube ausgehenden und zum Einführungsende 3 hin verlaufenden schlitzförmigen Spalt 5, wobei vorgesehen ist, dass der Schlitz während des Anbringens der Schraube im Gewebe und der Osseointegration mit dem Gewebe von einer lösbaren Abdeckleiste ausgefüllt wird.

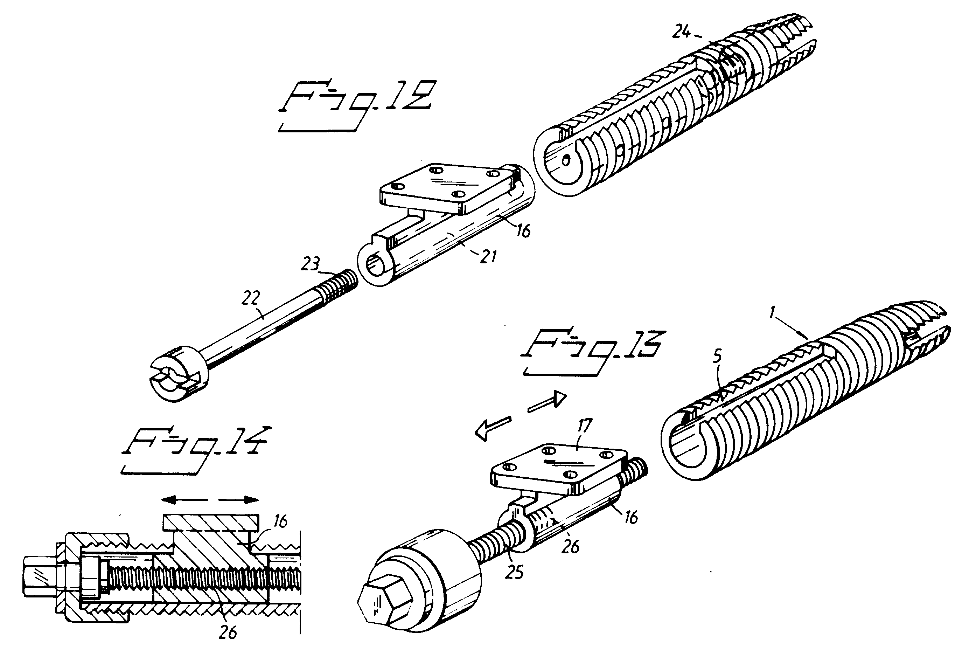

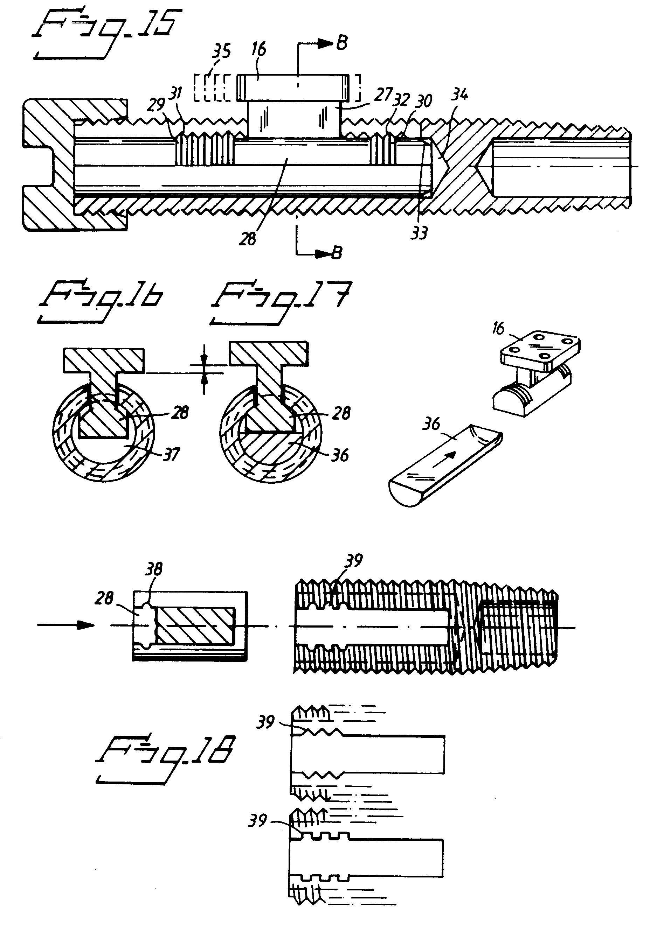

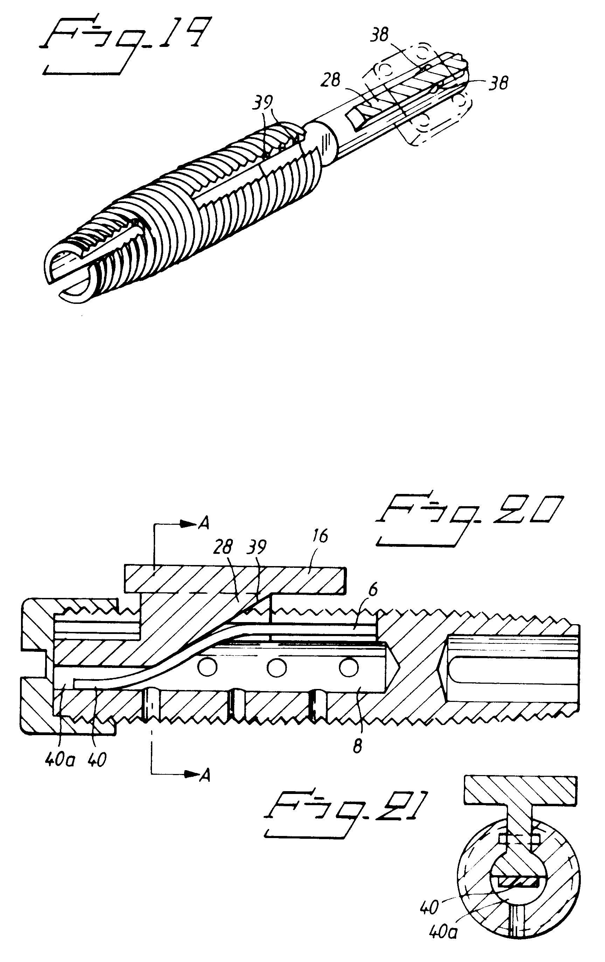

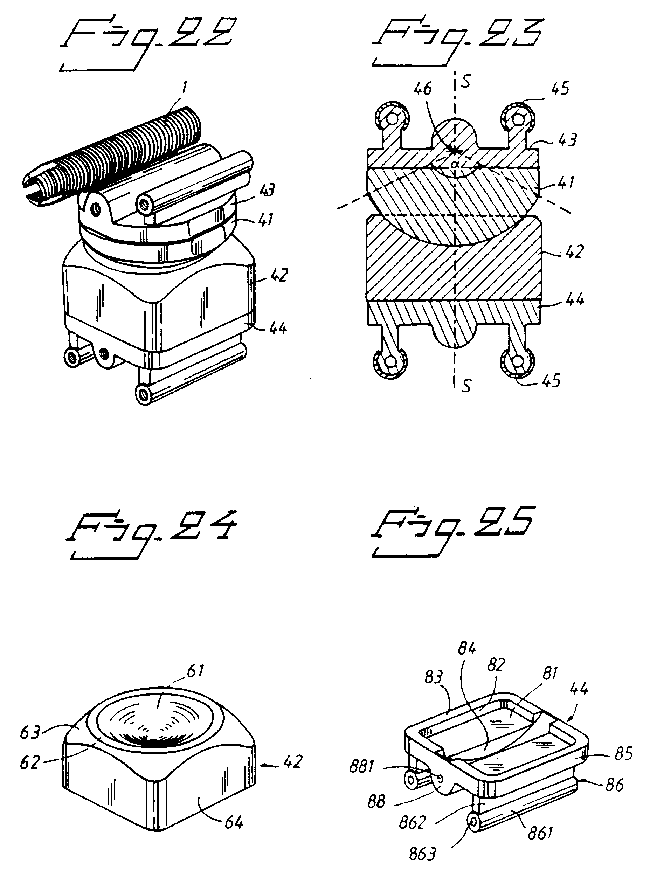

In Fig. 3 - 6 werden eine Anzahl geeigneter Ausführungen solcher Abdeckleisten 6 gezeigt, die mittels verschiedener Seitenprofile in diesen Profilen angepassten Ausbildungen der Seiten des Schlitzes 5 leicht eingeschoben und in ihrer Lage fixiert werden können. Zweckmässig ist die obere Fläche 7 der Abdeckleiste 6 etwas abgerundet und bildet dadurch einen natürlichen Übergang zwischen benachbarten Windungen des Aussengewindes 2. Die Formgebung der in Fig. 3 - 6 gezeigten Abeckleisten mit dazu angepassen Spaltquerschnitten ergeben eine gute Lagefixierung der Abdeckleisten im Spalt. Selbstverständlich kann die Abdeckleiste 6 auch in anderer Weise ausgebildet sein, z.B. mit geraden Seitenkanten und einem ebenen Bodenabschnitt zum Einschieben in den in Fig. 2 gezeigten, beispielsweise ausgefrästen Raum 5.

Die Abdeckleiste 6 sollte vorzugsweise den gesamten Spalt ausfüllen, d.h. die gleiche Länge wie der Spalt 5 aufweisen, sodass ein Hineinwachsen von Gewebe in den Spaltinnenraum während des Einheilens des Verankerungsorganes wirkungsvoll verhindert wird.

Das schraubenförmige Verankerungsorgan 1 besteht zweckmässig aus Titan oder weist wenigstens eine Titanbeschichtung auf. Zweckmässig ist ausserdem, dass die Oberfläche des Verankerungsorganes 1 zur Erzielung einer optimaler Osseointegration mit dem Gewebe mit Mikrodellen versehen ist, d.h. verhältnismässig dicht aneinander liegenden Dellen mit einem Durchmesser von zwischen 10 - 10.000 nm (Mikrodellen) und gegebenenfalls auch mit Makrodellen mit einem Durchmesser von 1 mµ bis zu 20 mµ und mehr. Hierbei können die Makrodellen zweckmässigerweise von Mikrodellen überlagert sein. Verschiedene technische Lösungen zur Herstellung solcher Titanoberflächen sind bekannt.

Die Abdeckleiste sollte zweckmässigerweise aus einem Material bestehen, das nicht im Gewebe verankert werden kann, und kann beispielsweise aus einem hierzu geeigneten Kunststoffmaterial bestehen oder auch aus einem blankpolierten Metallstreifen, der beispielsweise aus Titan besteht.

Grundgedanke vorliegender Erfindung ist dass der im Verankerungsorgan vorgesehene Spalt 5, 5a während des Einbringens des Organes in eine im voraus im Gewebe präparierte Bohrung von einer lösbar vorgesehenen Deckleiste 6, 6a abgedeckt werden soll, sodass ein Einwachsen des Gewebes in den Spalt verhindert wird und die Abdeckleiste in einer späteren Phase entfernt und durch einen in den so freigelegten Spalt einführbaren Halter für Prothesenteile, Anschlusselemente für Prothesen u.dgl. ersetzt werden kann.

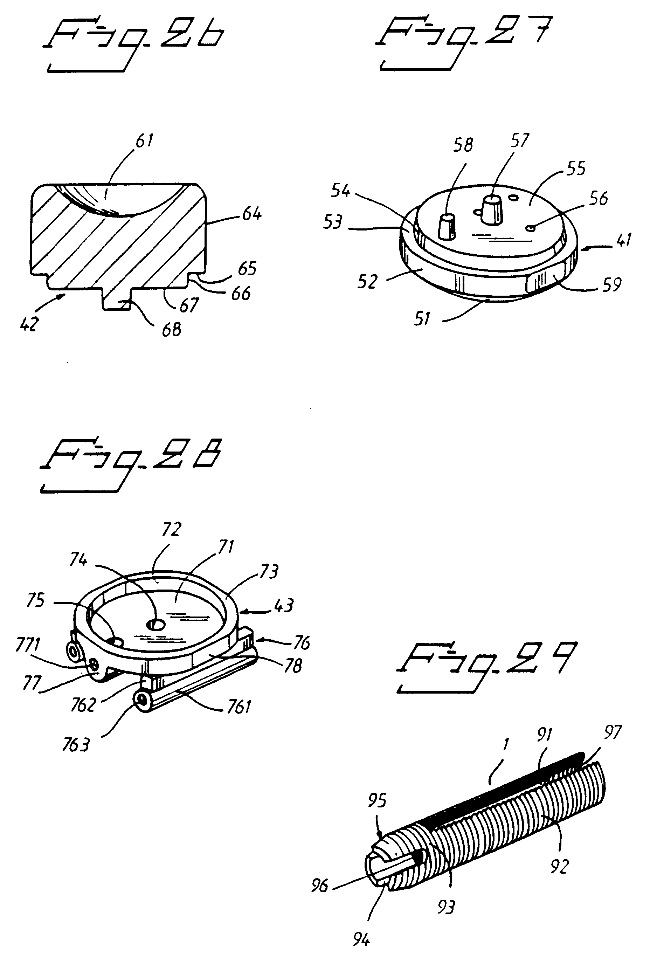

In Fig. 7 - 11 wird eine Weiterentwicklung des in Fig. 1 - 6 anschaulichten Grundprinzips gezeigt.

Wie aus Fig. 7 hervorgeht, weist das Verankerungsorgen 1 eine von seinem hinteren Ende 4 ausgehende Innernbohrung 8 auf, die mit dem Längsspalt 5 in Verbindung steht und zweckmässig etwas länger als der eigentliche Spalt 5 ist. Des sich konisch verjüngende Einführungsende 3 ist zweckmässig auch mit einer vom genannten Ende ausgehenden Innernbohrung 10 sowie mit vom Ende 3 ausgehenden Schlitzen 9 versehen, wobei letztere zur Erleichterung des Einschraubens der Schraube in die im Gewebe vorpräparierte Bohrung mit Schneidkanten 11 versehen ist. Die Innernbohrungen 8 und 9 stehen jedoch nicht miteinander in Verbindung. Darüber hinaus weist die Innenböhrung 8 eine Anzahl von Öffnungen 12 auf, die an der Aussenfläche der Schraube ausmünden. Diese Öffnungen 12 dienen zur Zufuhr von beispielsweise wachstumsfördernden oder entzündungshemmenden Mitteln in dem zwischen dem Gewebe und dem Aussengewinde befindlichen Hohlraum.

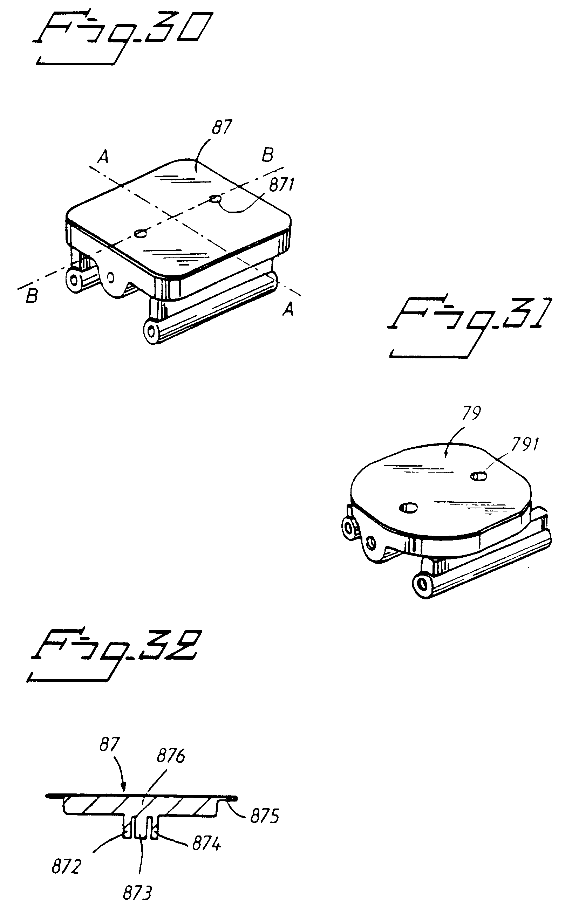

In Fig. 8 wird ein Mitnehmer 13 gezeigt, der in dieser alternativen Ausführung des Verankerungsorganes in die Innenbohrung 8 eingebracht werden soll und somit gleichzeitig zur Abdeckung des Spaltes mit einer von seiner Mantelfläche hervorstehender, den Abmessungen des Spaltes 5 angepasster nockenähnlicher Auskragung 14 dienen soll. In seinem vorderen Ende weist der Mitnehmer 13 einen Kreuzschlitz zum Zusammenwirken mit einem geeigneten, nicht gezeigten Instrument auf, wodurch des schraubenförmige Verankerungsorgan 1 leicht auf kontrollierbare Weise in seiner Lage fixiert und in die im Gewebe im Voraus angebrachte Bohrung eingeschraubt werden kann. Die nockenähnliche Auskragung 14 ergibt solchermassen eine zwangsgesteuerte Überführung von am Mitbringer 13 angreifenden Drehbewegungen auf die Schraube 1.

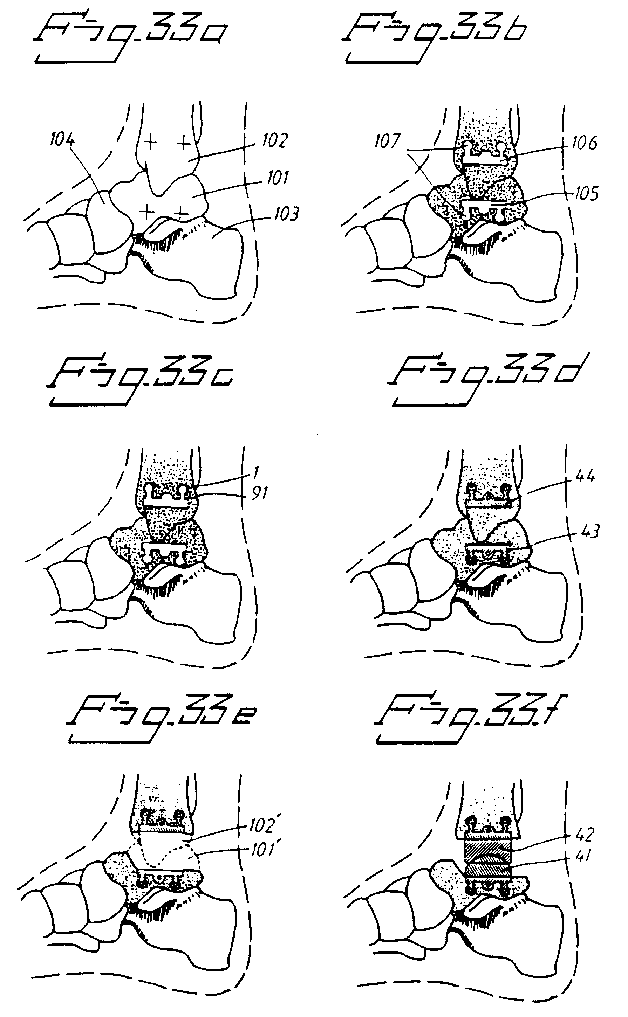

In Fig. 9 wird eine Ausbildung eines Halters 15 gezeigt, die in der Innenbohrung 8 und dem Spalt 5 angebracht werden soll, nachdem der Mitnehmer 13 nach Erzielung der Osseointegration der Schraube 1 mit dem Gewebe aus der Innenbohrung 8 entfernt worden ist. Hierbei hat der Halter 16 grundsätzlich die gleiche Formgebung wie der Mitnehmer, ist aber darüber hinaus mit einer auf der basisähnlichen Auskragung 14 vorgesehenen Grundplatte 17 zur Fixierung von Protheseteilen u.dgl. versehen.

In Fig. 10 wird der eine Teil des in Fig. 7 gezeigten Verankerungsorganes 1 mit Spalt 5, Innernbohrung 8 und dazu passendem, in Fig. 8 gezeigtem Halter 16 zum Anbringung im Spalt 5 gezeigt. Darüberhinaus wird eine mit Kreuzschlitz versehene Stoppschraube 18 zum Verschluss des oberen Endes 4 bei gleichzeitiger Lagesfixierung des Halters 16 gezeigt.

In Fig. 11 werden verschiedene Verriegelungen der Anschlagschraube 18 mittels aussen oder innen angebrachtem Gewinde gezeigt.

Eine solche Verriegelung des Mitnehmers oder Halters kann auch durch eine einfache Stoppschraube erzielt werden, die in ihrer Lage durch einen Körnerschlag fixiert wird.

Aus der in Fig. 12 gezeigten perspektivischen Darstellung mit aufgelösten Einzelteilen geht hervor, dass der Halter 16 eine durchgehende Bohrung 21 zur Aufnahme eines Bolzens 22 aufweist, dessen vorderer, gewindeversehener Abschnitt 23 mit einem entsprechenden Gewinde 24 im Boden der Innernbohrung 8 zusammenwirkt.

Bei der in Fig. 13 gezeigten Ausführung ist der Bolzenschaft 22 mit einem durchgehenden Gewinde 25 versehen, das sich über den grössten Teil des Bolzens erstreckt, wobei dieses Gewinde 25 mit einem entsprechenden Innengewinde 26 in der im Halter 16 vorgesehenen durchgehenden Bohrung 21 zusammenwirkt. Hierdurch kann man auf einfache Weise eine exakte Fixierung des Halters 16 entlang der Spalts 5 erzielen.

In Fig. 14 wird die in Fig. 13 veranschaulichte Vorrichtung in zusammengesetztem Zustand gezeigt.

In Fig. 15, 16 und 17 wird eine weitere Variante zur Lagesfixierung des Halters 16 gezeigt, die sich zur Feineinstellung des Halters in der Längsrichtung des Schlitzes eignet. Zu diesem Zweck weist der in der Innenbohrung 8 befindliche Halteteil 28, der diese Bohrung nur teilweise ausfüllt, auf seiner Obenseite wenigstens zonenweise vorgesehene Aussprünge 29, 30 auf, die mit entsprechenden Innennuten 31, 32 in der Innenbohrung 8 zusammenwirken. Hierbei ist zweckmässig der gegen das Einführungsende 3 gerichtete vordere Endabschnitt 33 des Halterteils 28 schräggestellt und vorgesehen gegen eine entsprechende Abschrägung 34 im Boden der Innenbohrung 8 anzuliegen. In dieser Weise kann der Halter 16 durch ein mit dem Teil 28 zusammenwirkendes keilförmiges Organ 36 leicht in den mit strichpunktierten Linien 35 angedeuteten Positionen arretiert werden, wobei die Vorsprünge 39 in die entsprechenden Nuten 31, 32 eingepresst werden.

Eine Lagefixierung des Halters 16 kann somit erzielt werden, wenn das in der Bohrung 8 befindliche Halterteil 28 beispielsweise wie in Fig. 16 ausgebildet ist, d.h. die Innenbohrung 8 nur teilweise ausfüllt, wobei eine optimale Feineinstellen 9 dadurch erzielt werden kann, dass der in Fig. 17 gezeigte Keil 36 in den vom Halterteil 28 nicht ausgefüllten unteren Hohlraum 37 der Innenbohrung 8 eingetrieben wird.

Ein weiteres, in den Zeichnungen nicht gezeigtes Verfahren zur Lagefixierung des Halters 16 im Spalt 5 kann durch geeignete Passtücke erzielt werden, die in die Innenbohrung 8 vor und hinter einem Halter 16 in einer kürzeren Ausführung als normal eingeschoben werden, beispielsweise in einer im Zusammenhang mit Fig. 17 gezeigten Ausführung des Halters 16.

In Fig. 18 werden schematisch einige weitere Ausführungen einer geeigneten Lagesfixierung des Halters 16 gezeigt. Wie ersichtlich, kann das Halterteil 28 beispielsweise mit auf verschiedenste Weise ausgebildeten, von dessen Mantelfläche hervorragenden Vorsprüngen 38 versehen sein, die zum Zusammenwirken mit entsprechenden, als Ausnehmungen ausgebildeten Einkerbungen 39 längs der Langseiten des Spalts 5 ausgebildet sind.

Bei der in Fig. 19 gezeigten Ausführung wurde der vom Halterteil 28 herausragende und den Spalt 5 zumindest teilweise ausfüllende nockenartige Absatz mit solchen Vorsprüngen 38 versehen, die mit längs des Spalts 5 vorgesehenen seitlich ausgebildeten Einkerbungen 39 zusammenwirken.

Bei der in Fig. 20 und 21 gezeigten Ausführungsform wurde der Spalt 5 ursprünglich von einer etwas flexiblen, verhältnissmässig dünnen, streifenförmigen Abdeckleiste 6 abgeschirmt, die vor dem Einführen der Halterung 16 nicht entfernt werden muss. Hierbei ist das vordere Halterteil 28 des Halters 16 mit einer schräggestellten Fläche 39 ausgebildet, mittels der die Abdeckleiste 6 während des Einführens des Halters 16 in die Innenbohrung 8 automatisch in Richtung zur Innenbohrung 8 abgebogen wird. Hierbei wird der Halterteil 28 zweckmässig nur den oberen, nahe am Spalt 5 gelegenen Teil der Innenbohrung 8 ausfüllen. Dadurch kann der abgebogene Teil 40 der Abdeckleiste 6 im freien Hohlraum 40a aufgenommen werden. Durch diese Ausführungsform wird eine indirekte Lagefixierung der Halters 16 erzielt, während der nicht ausgenützte Teil des Spaltes 5 fortwährend von der Abdeckleiste 6 abgeschirmt ist.

In Fig. 22 und 23 wird in perspektivischer Darstellung, von unten her, ein künstliches Fussgelenk gezeigt, dass aus einem hochglanzpolierten Taluskugelglied 41 aus legiertem Titan (Ti6A14V) einem Tibiaschalenelement 42 aus HD-Polyethylen, einem das Taluskugelglied 41 teilweise umschliessenden Talusimplantatkörper 43 und einem das Tibiaschalenglied 42 teilweise umschliessenden Tibiaimplantatkörper 44 besteht. Die Glieder 41 - 44 werden in Fig. 22 und 23 in der "geraden" oder "gestreckten" Anordnung des Glieds, d.h. beim grössten gegenseitigen Abstand von Talusimplantatkörper 43 und Tibiaimplantatkörper 44 gezeigt, und die Glieder 41 - 44 sind um die fluchtend angeordneten Gelenkachsen (S - S) zentriert. Das Tibiaschalenglied 42 hat an seiner dem Taluskugelglied 41 zugekehrten Seite die Form einer sphärischen Kalotte mit einem Kalottenwinkel von etwa 130°, d.h. einer sogenannten Taluskugel 51 (Fig. 27). Die Taluskugel ist formkongruent mit eine negativ sphärischen kalottenförmigen Vertiefung im Tibiaschalenglied 42, der sogenannten Tibiaschale 61. Im montierten Zustand des künstlichen Fussgelenkes stützt sich die Taluskugel 51 in der Tibiaschale 61 ab und ist unter Anliegung frei in dieser bewegbar.

Das Tibiaschalenelement 42 hat die Form eines Parallelepipeds mit quadratischer, an den Ecken abgerundeter Grundform. Die Grundform, d.h. der von der Tibiaschale 61 abgewandte Teil des Tibiaschalenglied 42 ist im wesentlichen formkongruent mit der dem Gelenk zugekehrten Seite des Tibiaimplantatkörpers 44 (Fig. 25).

Der Tibiaimplantatkörper 44 hat die Form eines niedrigen Parallelepipeds mit demselben quadratischen Grundquerschnitt wie das Tibiaschalenglied 42 und mit gleichsam abgerundeten Ecken, sodass die Seiten 64, 85 des Tibiaschalengliedes 42 und des Tibiaimplantatkörpers 44 in gleicher Ebene liegen und ineinander übergehen. Die dem Tibiaschalenglied 42 zugekehrte Seite des Tibiaimplantatkörpers 44 hat eine etwa quadratische Ausnehmung mit abgerundeten Ecken und einem ebenen Boden 81 und etwas nach innen geneigten Innenwänden.

Der Boden 81 und die gegenüberliegenden Seiten der umlaufenden Wand der Ausnehmung sind mit einem, in einer zum Boden 81 senkrechten Richtung sich erstreckenden, rechteckigen Einschnitt versehen, der sich zwischen gegenüberliegenden Aussenseiten an der umlaufenden flachen Krone 83 der Wand der Ausnehmung ausdehnt und der in Seitenansicht die Form eines Kreissegments hat; vgl. die Seitenwand 84 des Einschnitts.

Im montiertem Zustand des Tibiaschalenglieds 42 auf dem Tibiaimplantatkörper 44 liegt dessen umlaufende, einen Absatz bildende (Fig. 26) Grundkante 65 gegen die flache Wandoberseite 83, die Grundfläche 67 gegen den Boden 81 und die in Richtung zur Grundfläche 67 leicht geneigte Absatzwand 66 gegen die gleichsam geneigte Innenwand 82 der Ausnehmung an. Ein in Seitenansicht halbmondförmiger Rücken 68, der die Grundfläche 67 in zwei gleiche, im wesentlichen rechteckige, Abschnitte unterteilt, erhebt sich über die Grundfläche 67 und hat Passform gegenüber dem kreissegmentförmigen Einschnitt im Tibiaimplantatkörper 44. Der Abstand zwischen der von der umlaufenden Wandkrone 83 definierten Ebene und dem Mittelpunkt des kreissegmentförmigen Einschnitts ist wohl grösser als die Höhe des Tibiaimplantatkörpers 44, wird aber durch einen in seinem Querschnitt halbkreisförmigen, mit dem Tibiaimplantatkörper einstückig verbundenen Mittelrücken 88 ermöglicht, der die Materialstärke des Tibiaimplantatkörpers 44 im Bereich des genannten Einschnitts erhöht. Der Mittelrücken 88 verläuft parallel mit zwei gegenüberliegenden Aussenseiten 85 des Tibiaimplantatkörpers und erstreckt sich zwischen den beiden anderen gegenüberliegenden Aussenseiten des Implantatkörpers. Der Mittelrücken 88 ist an seinen beiden Enden mit kurzen, nicht durchgehenden Bohrungen 88 zum Eingriff von Werkzeugen und dgl. versehen.

Parallel zum Mittelrücken 88 und zu dessen beiden Seiten der Tibiaimplantatkörper 44 liegen zwei mit diesem einstückige Seitenrücken 86 vor. Die Seitenrücken 86, deren Höhe grösser als die des Mittelrückens 88 ist, haben über ihre gesamte Länge einen schmaleren Rückenfuss 862 und einen breiteren Rückenkopf 861, der im Schnitt senkrecht zur Längenausdehnung der Seitenrücken 86 die Form eines Kreissegments mit einem Segmentwinkel von mehr als 270° hat. An beiden Enden der Rückenköpfe 861 sind Mittelbohrungen 863 angebracht, die mit Innengewinden versehen sind.

Der Talusimplantatkörper 43 hat die Form eines zylindrischen Körpers von geringer Höhe, der aus einem Boden 71 und einem Mantel mit Aussenfläche, Innenfläche 72 und einem planen, ringförmigen Mantelrand 73 besteht. Die Innenfläche 72 des Mantels ist schwach gegen den Boden geneigt (d.h. hat die Form eines Kegelstumpfes), während die Aussenfläche vier gegeneinander um 90° versetzte, symmetrisch angeordnete abgeplattete Bereiche 78 aufweist. Der Boden 71 ist mit zwei Bohrungen 74, 75 versehen, die sich in einem mit dem Boden 71 einstückig verbundenen Mittelrücken fortsetzen, der sich zwischen den Mittelpunkten zweier gegenüberliegender abgeplanten Bereiche an der Aussenfläche des Mantels ausdehnt. Der Mittelrücken ist an seinen beiden Enden mit nicht durchgehenden Bohrungen 771 versehen.

Parallel zum Mittelrücken 77 des Talusimplantatkörpers und zu dessen beiden Seiten hat der Talusimplantatkörper 77 zwei einstückig verbundene Seitenrücken. Die Seitenrücken 76 von grösserer Höhe als der Mittelrücken 77 haben über ihre gesamte Länge einen schmaleren Rückenfuss 762 und einen breiteren Rückenkopf 761, der im Schnitt senkrecht zu der Längsausdehnung der Seitenrücken 76 die Form eines Kreissegmentes mit einem Segmentwinkel von ca. 270° hat. In den beiden Endbereichen der Rückenköpfe 761 sind Mittelbohrungen 763 angebracht, die mit Innengewinden für den Werkzeugeingriff und dgl. versehen sind.

Das Taluskugelelement 41 hat die Form eines zylindrischen Körpers von geringer Höhe, der einstückig mit der Taluskugel 51 verbunden ist und dessen Mantel 52 von dieser geschnitten wird. Der Zylindermantel 52 weist zwei einander diametral entgegengesetzte abgeplante Bereiche 58 auf und geht in einen ebenen, ringförmigen Absatz 53 uber, von dem ein kegelstumpfförmiger Abschnitt mit vom Absatz 53 aus schwach nach innen geneigter Seitenwand 54 hervorsteht. Der kegelstumpfförmige Abschnitt hat eine kreisförmige ebene Oberfläche 55, die zwei auf einen Durchmesser angeordnete Zapfen 57, 58 aufweist, wobei letztere sich kegelförmig in Richtung ihrer freien Enden verjüngen und von denen der eine Zapfen 57 im Mittelpunkt der Fläche 55 angeordnet ist. Die ebenen Bereiche 58 am Zylindermantel 52 verlaufen parallel zu dem durch die Zapfen 57, 58 gezogenen Durchmesser.

Im montierten Zustand des Taluskugelglieds 41 auf dem Talusimplantatkörper 43 liegt dessen umlaufende, einen Absatz (Fig. 27) bildende Grundkante 53 gegen den Zylindermantelrand 73, die Fläche 55 gegen den Boden 71 sowie die in Richtung zur Fläche 55 schwach nach innen geneigte Absatzwand 54 gegen die entsprechend geneigte Zylinderinnenwand 72 an. Im Boden 71 des Talusimplantatkörpers 43 sind zwei nicht durchgehende zylindrische Bohrungen 74, 75 angebracht, deren Lage und Dimension den schwach konischen Zapfen 57, 58 des Taluskugelglieds 41 entsprechen. Die Bohrungen 74, 75 durchdringen nicht den verhältnismässig dünnen Boden 75, da ein in ihrem Querschnitt halbkreisförmiger, mit dem Boden 71 des Talusimplantatkörpers 71 an dessen Aussenseite einstückig verbundenen Mittelrücken die Materialdicke des Bodens 71 im Bereich der genannte Bohrungen 791 erhöht. Der Mittelrücken 77 ist in seinen beiden Enden mit kurzen, Innengewinde aufweisenden Bohrungen 771 versehen und dehnt sich parallel zu den zwei einander gegenüberliegenden ebenen Bereichen 78 an der Zylindermantelaussenwand des Talusimplantatkörpers 43 aus.

Parallel mit dem Mittelrücken 77 des Talusimplantatkörpers 43 und zu dessen beiden Seiten sind zwei Aussenrücken 76 mit dem Talusimplantatkörper 43 einstückig verbunden. Die Aussenrücken 76, welche die Aussenseite des Bodens 71 starker überragen als der Mittelrücken 77, haben über ihre gesamte Länge einen schmaleren Rückenfuss 762 und einen breiteren Rückenkopf 761, der im Schnitt senkrecht zur Längenausdehnung der Aussenrücken 76 die Form eines Kreissegments mit einem Segmentwinkel von ca. 270° aufweist. An den beiden Enden der Rückenköpfe 761 sind Mittelbohrungen 763 angebracht, die mit Innengewinde versehen sind. Die ebenen Bereiche 58 legen zwei Ebenen fest, welche die Aussenflächen der zylinderförmigen Rückenköpfe 761 tangential in deren gedachten, voneinander den grössten Abstand aufweisenden langen Begrenzungslinien berühren.

Die flachen Aussparungen des Talusimplantatkörpers 43 und des Tibiaimplantatkörpers 44 in Richtung zum Taluskugelglied 41 bzw. das Tibiaschalenglied 42 können mit jeweils einer Kappe 87, 79 verschlossen werden. Die aufgesetzte Taluskappe 79 überdeckt die zur Aufnahme der Taluskugel 41 ausgebildete Vorderseite vollständig und liegt Kante-an-Kante mit der Aussenkontur des planen Rings 73, der das freie Ende des Zylindermantels (Fig. 28, 32) bildet und ist mit zwei nicht durchgehenden Bohrungen 791 zum Eingriff von Werkzeugen bei Montage und Demontage versehen. Die nicht gezeigte Innenseite der Taluskappe 79 stimmt in ihrer Form mit der in Fig. 27 gezeigten "Innenseite" des Taluskugelglieds 41 überein, das nach Montage am Talusimplantatkörper 43 an diesem anliegt. Die Taluskappe 79 weist somit den Wand- und anderen Gliedern 53, 54, 55, 57 und 58 des Talusimplantatkörpers 43 entsprechende Teile auf.

Auf gleiche Weise schützt die aufgesetzte Tibiakappe 87 die Aussparung des Tibiaimplantatkörpers 44 (Fig. 30). Die zwei in der Aussenseite angebrachten, nicht durchgehenden Bohrungen 871 sind für den Eingriff von Werkzeugen vorgesehen. In Fig. 31 wird die Tibiakappe 87 im Schnitt gezeigt. Deren umlaufende Ringkante 875 von kleiner Materialstärke liegt gegen die Wandkrone 83 am Tibiaimplantatkörper an. Die Materialstärke im zentralen Bereich 876 der Kappe 87 ist der Tiefe der im wesentlichen quadratischen Aussparung im Tibiaimplantatkörper 44 angepasst. Drei parallele, in Seitenansicht halbmondförmige Rippen 872, 873, 874 sind an der Innenseite der Tibiakappe 87 auf solche Weise vorgesehen, dass sie von dem kreissegmentförmigen Einschnitt im Boden 81 des Tibiaimplantatkörpers 44 aufgenommen werden. Die äusseren Rippen 872, 873 sind dünner und etwas federnd ausgebildet, um die Kappe zu fixieren.

Die zylindersektionsförmigen, in der Längsrichtung des Implantatkörpers 43 bzw. des Tibiaimplantatkörpers 44 verlaufenden Rückenköpfe 761 und 861 lassen sich in die mit Innenbohrung und Aussengewinde versehenen Verankerungsschrauben 45 einführen, wobei letztere einen in ihrer Längsrichtung verlaufenden offenen Schlitz 91 aufweisen, dessen Breite etwas grösser ist als die Breite der in Längsrichtung der Fussabschnitte 762 bzw 862 verlaufenden Seitenrücken und deren offenes Ende als Einführungsende bezeichnet wird. In Fig. 22 wird eine Verankerungsschraube 1 gezeigt, in die der eine der Seitenrücken 76 des Talusimplantatkörpers 43 eingeführt ist. Die Innenbohrung (Fig. 29) der Verankerungsschraube ist nicht durchgehend, sondern wird durch eine etwa zwei Windungen des Aussengewindes dicke Querwand 96 in einen längeren und einen kürzeren Abschnitt aufgeteilt; im Bereich der Querwand 96 ist das Aussengewinde der Verankerungsschraube 1 somit nicht unterbrochen. Der Schlitz 91 erstreckt sich über die gleiche Länge wie der lange Abschnitt der Innenbohrung, während der kurze Abschnitt der Innenbohrung an seinem sogenannten Einführende eine sich verjüngende Zone 95 sowie drei symmetrisch angeordnete, sich in Längsrichtung der Schraube erstreckende und gegen das Einschraubende hin offene Schlitze 94 hat. Das Aussengewinde der Verankerungsschraube 1 ist selbstschneidend. Am Einrührende ist die Verankerungsschraube mit einem kurzen Innengewinde 97 versehen.

Im folgenden wird eine geeignete Ausführungsform zur Rekonstruktion eines beschädigten Fussgelenks mittels einer erfindungsgemäss vorgeschlagenen Verankerungsvorrichtung naher beschrieben werden, wobei auf Fig. 33a -33 f verwiesen wird.

In Fig. 33 wird grundsätzlich das Skelett des hinteren Teils eines Fusses in medialer Sicht gezeigt, wobei 101 den Talus, 102 die Tibia, 103 das Fersenbein und 104 das Kahnbein bezeichnet. Der Talus 101, das distale Ende der Tibia sowie der oberste Teil des Fersenbeins 103 werden medial freigelegt und mit Hilfe einer Schablone werden die vier mit + bezeichneten Punkte (Fig. 33a) markiert, die die Ecken eines Rechtecks ausmachen. In diesen Punkten werden senkrecht (medial/lateral) vier Bohrungen 107 (Fig. 33b) angebracht, mit einer der Verankerungsschraube 1 entsprechenden Länge. Im Anschluss daran wird, ausgehend von den beiden Bohrungen 107 im Talus, der mit 105 bezeichnet Hohlraum freipräpariert und ein entsprechender, mit 106 bezeichneter Hohlraum wird, ausgehend von den beiden Bohrungen 107 in der Tibia 102, freipräpariert. Gesehen in Richtung der sich in Längsrichtung erstreckenden Rücken 76, 77 sowie 86, 88 entsprechen die Konturen der Hohlräume 105 und 107 denen des Talusimplantatglieds 43 bzw. des Tibiaimplantatglieds 44, wobei die Bohrungen 107 einen Durchmesser erhalten, der ein selbstschneidendes Einschrauben der Verankerungsschrauben 1 ermöglicht. Dieses Einschrauben führt zu dem in Fig. 33c angegebenen Resultat, nämlich, dass das Einschrauben beendet wird, wenn die Schrauben 1 (innerhalb einer Ganghöhe) die korrekte Einschraublänge erreicht haben, wobei die Position der Schlitze so eingestellt wird, dass sie sich paarweise (Schraubenpaar Talus/Tibia) in aufeinander zugerichteter Lage befinden.

Danach werden der mit der Abdeckkappe 79 versehene Talusimplantatkörper 43 und der mit der Abdeckkappe 87 versehene Tibiaimplantatkörper 44 mit ihren Rücken in Flucht mit dem jeweiligen Paar von Verankerungsschrauben 1 (Schraubenpaar fur Talus bzw. Tibia) und deren Schlitzen 91 gebracht und in die Verankerungsschrauben 1 und die im Talus 101 bzw. in der Tibia 102 freipräparierten Hohlräume 105, 106 (Fig. 33d) eingeführt. Die Einführungstiefe kan mittels hier nicht gezeigter verstellbarer Abstandsschrauben eingestellt werden, wobei letztere in den Bohrungen 763, 863 an den lateral ausgerichteten Enden der Seitenrücken 76, 86 angebracht werden und in montiertem Zustand gegen die Zwischenwand 96 der Verankerungsschrauben 1 anliegen. Der Talusimplantatkörper 43 und der Tibiaimplantatkörper 44 werden in ihrer eingeführten Lage durch nicht gezeigte kurze Anschlagschrauben arretiert, wobei letztere in das Innengewinde 97 der Verankerungsschrauben 1 bis zum Anliegen an die medial ausgerichteten Enden der Seitenrücken 76, 86 eingeschraubt werden.

Die Operationswunde wird verschlossen und nach kurzer Heilungsdauer kann der Patient das an sich beschädigte Fussgelenk weiter verwenden. Nach einigen Monate sind die einoperierten Verankerungsschrauben 1 mit dem Talusimplantatkörper 43 und dem Tibiaimplantatkörper 44 integriert und im Knochengewebe verankert, s.g. Osseointegration. Danach wird der Patient zum zweiten Male operiert. Dabei werden die in Fig. 33e mit gestrichelter Linie angedeuteten Teile 101', 102' des Talus bzw. der Tibia durch die Taluskappe 79 und die Tibiakappe 87 entfernt, worauf die genannten Kappen 79, 87 entfernt werden. Dann werden das Taluskugelglied 41 und das Tibiaschalenglied 42 in die jeweilige Ausnehmung am Talusimplantatkörper 43 und am Tibiaimplantatkörper 44 eingesetzt und die Wunde wird verschlossen.

Das Künstliche Fussgelenk 41, 42 ist nun nach beendetem chirurgischen Eingriff direkt belastbar. Durch dieses Zweistufenverfahren kann der Patient während des Einwachsens der Verankerungselemente mit dem an sich beschädigten Fussgelenk einigermassen auskommen, bis zur endgültigen Einsetzung des künstlichen Fussgelenkes.

Ein künstliches Fussgelenk kann erfindungsgemäss auch derart verankert werden, dass im Talus und/oder in der Tibia in einer im Verhältnis zu der in Fig. 33a um 90° gedrehten Richtung Bohrungen angebracht werden, nämlich dorsal/anterioral. In diesem Fall kann es notwendig sein, einen kleineren Teil des obersten Talusmittelteils zu entfernen.

Auf ähnliche Weise können auch andere Gelenke ganz oder teilweise ersetzt werden. Beispielsweise eignet sich die erfindungsgemässe Verankerungsvorrichtung zur Verankerung von Kniegelenkprothesen, Hüftgelenkprothesen, Achselgelenkprothesen, Ellenbogengelenkprothesen, Handgelenkprothesen, usw.

## Patentansprüche

1. Zur Implantation in Geweben vorgesehenes, hauptsächlich rotationssymmetrisch ausgebildetes und aus einem gewebeverträglichen Material bestehendes Verankerungsorgan (1) zur Halterung von Prothesen, künstlichen Gelenkkomponenten u.dgl., wobei das Verankerungsorgan ein von seinem hinteren Ende (4) ausgehendes und in Richtung zum Einführungsende (3) verlaufendes Aussengewinde (2) aufweist, und dass wenigstens ein in der mit dem Aussengewinde (2) versehenen Mantelfläche in Abstand vom Einführungsende (3) angeordneter schlitzförmiger Spalt (5, 5a) vorgesehen ist, dadurch **gekennzeichnet**, dass der schlitzförmige Spalt (5, 5a) vom hinteren Ende des Verankerungsorganes (1) ausgeht, hauptsächlich axial verläuft, und zur lösbaren Aufnahme eines den schlitzförmigen Spalt (5, 5a) ausfüllenden Abdeckgliedes während des Einschraubens des Verankerungsorgans in eine im Gewebe vorpräparierten Ausnehmung vorgesehen ist.

2. Verankerungsorgan nach Anspruch 1, dadurch **gekennzeichnet**, dass der genannte Spalt (5, 5a) während des Anbringens des Verankerungsorganes (1) in eine im Gewebe vorpräparierte Ausnehmung und während der Osseointegrationsperiode mit einer den Spalt (5, 5a) abschirmenden, lösbaren Leiste (6, 6a) versehen ist.

3. Verankerungsorgan nach Anspruch 1 oder 2, dadurch **gekennzeichnet**, dass der Spalt (5, 5a) nach dem Entfernen der Abdeckleiste (6, 6a) zur lagefixierbaren Aufnahme der genannten Prothesenteile, künstlichen Gelenke und Gelenkteile u.dgl., vorgesehen ist.

4. Verankerungsorgan nach Anspruch 1 - 3, dadurch **gekennzeichnet**, dass der Spalt (5, 5a) eine in das Verankerungsorgan gefräste Nut ist, deren Begrenzungskanten zur lösbaren Fixierung der Abdeckleiste (6, 6a) vorgesehen sind.

5. Verankerungsorgan nach Anspruch 1 - 4, **gekennzeichnet**, durch eine vom hinteren Ende (4) des Verankerungsorgans (1) ausgehende Innenbohrung (8), die mit dem genannten Spalt (5, 5a) in Verbindung steht.

6. Verankerungsorgan nach Anspruch 5, dadurch **gekennzeichnet**, dass die Länge der Innenbohrung etwas grösser als die Länge des mit dieser zusammenwirkenden schlitzförmigen Spaltes (5, 5a) ist.

7. Verankerungsorgan nach Anspruch 5 oder 6, dadurch **gekennzeichnet**, dass das Abdeckglied (6, 6a) aus einem Mitnehmer (13) besteht, der mittels mindestens einer axialen Auskragung (14) zum Einschieben und Abdecken des genannten spaltförmigen Schlitzes (5, 5a) unter zumindest teilweisem Ausfüllen der Innenbohrung vorgesehen ist, und welcher so ausgebildet ist, dass bei Umdrehung des Mitnehmers das Verankerungsorgan mit dreht.

8. Verankerungsorgan nach Anspruch 7, dadurch **gekennzeichnet**, dass der schlitzförmige Spalt (5) mit an seinen Längsseiten angeordneten und als Ausnehmungen ausgebildeten Einkerbungen (39) zur Lagefixierung des Mitnehmers (13) oder zur Lagefixierung eines in den Spalt eingeschobenen Halters (16) zur Aufnahme von Prothesenelementen, u.dgl. ausgebildet ist, wobei der Durchmesser der Innenbohrung (8) grösser ist als die Breite des Spaltes (5, 5a) senkrecht zur Längsachse der Innenbohrung gemessen.

9. Verankerungsorgan nach Anspruch 8, dadurch **gekennzeichnet**, dass der Mitnehmer (13) nach Einführung des Verankerungsorgans (1) im Gewebe gegen einen Halter (16) von grundsätzlich gleicher Ausbildung austauschbar ist, wobei der Halter (16) mit einer an der Auskragung (14) angeordneten Grundplatte (17) zur Halterung von Prothesenteilen u.dgl. versehen ist.

10. Verankerungsorgan nach Anspruch 9, dadurch **gekennzeichnet**, dass das hintere Ende des Verankerungsorganes (1) nach dem Einführen des Halters (16) in die Innenbohrung (8) durch eine Stoppschraube (18) bei gleichzeitiger Lagefixierung des Halters (16) verschliessbar ist.

11. Verankerungsorgan nach Anspruch 9 und 10, dadurch **gekennzeichnet**, dass der Halter (16) eine durchgehende Bohrung (21) zur Aufnahme eines Bolzens (22) aufweist, dessen vorderer Gewindeabschnitt mit einem entsprechenden Gewinde (24) im Boden der Innenbohrung (8) zusammenwirkt.

12. Verankerungsorgan nach Anspruch 11, dadurch **gekennzeichnet**, dass der Bolzen (22) ein Aussengewinde (25) zwecks einer Lagefixierung des Halters (16) zum Zusammenwirken mit einem entsprechenden Innengewinde (26) in der im Halter (16) angeordneten Bohrung (21) aufweist.

13. Verankerungsorgan nach einem der Ansprüche 9-12, dadurch **gekennzeichnet**, dass der Halter (16) mit einem Teil (28) wenigstens teilweise die Bohrung (8) ausfüllt, wobei der Teil (28) auf seiner Oberseite wenigstens zonenweise Vorsprünge (29, 30) aufweist, die zwecks einer Lagefixierung des Halters (16) mit in der Innenbohrung (8) vorhandenen Einkerbungen (31, 32) zusammenwirken.

14. Verankerungsorgan nach einem der Ansprüche 9-13, dadurch **gekennzeichnet**, dass der in der Innenbohrung (8) befindliche Teil (28) des Halters (16) diese nur teilweise ausfüllt und zwecks einer Lagefixierung des Halters (16) mittels keilförmiger Einsatzelemente (36) mit seinen Vorsprüngen (29, 30) in Eingriff mit den zuständigen Einkerbungen bringbar ist.

15. Verankerungsorgan nach einem der Ansprüche 9-14, dadurch **gekennzeichnet**, dass der in der Innenbohrung (8) vorhandene Teil des Halters (16) Vorsprünge (38) zur Erzielung einer Lagefixierung des Halters (18) mit entsprechenden Ausnehmungen (39) entlang den Langseitenwänden des Spaltes (5) aufweist.

16. Verankerungsorgan nach einem der Ansprüche 9-15, dadurch **gekennzeichnet**, dass die Abdeckleiste (6) aus einer flexiblen, verhältnismässig dünnen, streifenförmigen Abdeckleiste (6) besteht, die nach und nach in einen zwischen Halter (16) und Innenbohrung (8) vorhandenen freien Raum beim Einschieben des Halters (16) in den Spalt (5) einpressbar ist.

17. Verankerungsorgan nach einem der vorhergehenden Patentansprüche, dadurch **gekennzeichnet**, dass das Einführungsende (3) des Verankerungsorganes (1) sich konisch verjüngt und einen oder mehrere mit Schneidkanten versehene Schlitze (9) aufweist, die vom offenen Bodenabschnitt des Verankerungsorganes (1) ausgehen.

18. Vorrichtung zur Verankerung einer Gelenkprothese u.dgl. im Knochengewebe bestehend aus wenigstens einem zur Implantation im Gewebe vorgesehenen, hauptsächlich rotationssymmetrisch ausgebildeten und aus einem gewebeverträglichen Material bestehenden Verankerungsorgan (1) und wenigstens einer für die Gelenkprothese vorgesehenen Halterung (16), wobei das Verankerungsorgan ein von seinem hinteren Ende (4) ausgehendes und in Richtung zum Einführungsende (3) verlaufendes Aussengewinde (2) aufweist, dadurch **gekennzeichnet**, dass
- das Verankerungsorgan (1) wenigstens einen, in der gewindeversehenen Manteloberfläche im Abstand vom Einführungsende (3) angeordneten, schlitzförmigen Spalt (5, 5a) aufweist, welcher vom hinteren Ende des Verankerungsorganes ausgeht, hauptsächlich axial verläuft, und zur lösbaren Aufnahme eines den Schlitz ausfüllenden Abdeckgliedes während des Einschraubens des Verankerungsorgans in eine im Gewebe vorpräparierten Bohrung vorgesehen ist, dass das Verankerungsorgan (1) zum Einschrauben in die dicht neben einem Gelenk und im wesentlichen parallel zu der Gelenkachse oder der Gelenkebene im Knochengewebe vorpräparierte Bohrung vorgesehen ist, und dass
- das Verankerungsorgan (1) über den schlitzförmigen Spalt (5, 5a) zur lösbaren Aufnahme der für die Gelenkprothese vorgesehenen Halterung (16) vorgesehen ist.

19. Verankerungsvorrichtung nach Anspruch 20, dadurch **gekennzeichnet**, dass sie zwei oder mehrere Verankerungsorgane (1) umfasst.

## Claims

1. A mainly axially symmetrical anchoring member 1, made from a tissue-compatible material and provided for implantation in tissues, for the retention of prostheses, artificial joints and the like, the anchoring member having an external threading 2 starting from a rear end 4 and extending in the direction of an introduction end 3, and at least one slot-shaped gap 5 being provided at a distance from the introduction end 3 in the surface formed with the external threading 2, characterised in that the slot-shaped gap 5, 5a of the anchoring member 1 starts from the rear end, extends mainly axially, and is provided for the releasable reception of a covering member filling the slot-shaped gap 5, 5a while the anchoring member is being screwed into a recess previously prepared in the tissue.

2. An anchoring member according to Claim 1, characterised in that during the application of the anchoring member 1 in a recess previously prepared in the tissue and during the osseointegration period, said anchoring member 1 is provided with a releasable strip-shaped cover 6, 6a which screens the gap 5, 5a.

3. An anchoring member according to Claim 1 or 2, characterised in that after the covering strip 6, 6a has been removed, the gap 5, 5a is provided for the reception of said prosthesis parts, artificial joints and parts of joints and the like in a fixable position.

4. An anchoring member according to Claims 1-3, characterised in that the gap 5, 5a is a groove which is milled in the anchoring member and whose boundary edges are provided for the releasable fixation of the covering strip 6, 6a.

5. An anchoring member according to Claims 1-4, characterised by an internal bore 8 which starts from the rear end 4 of the anchoring member 1 and is connected to said gap 5, 5a.

6. An anchoring member according to Claim 5, characterised in that the length of the internal bore is slightly greater than the length of the slot-shaped gap 5, 5a cooperating therewith.

7. An anchoring member according to Claim 5 or 6, characterised in that the covering strip 6, 6a consists of an entraining member 13 which is adapted by means of at least one axial projection 14 to be inserted and cover said slot-shaped gap 5, 5a, while at least partially filling the internal bore, and which is so constructed that when the entraining member rotates, the anchoring member makes a corresponding rotation.

8. An anchoring member according to Claim 7, characterised in that the slot-shaped gap 5 is constructed with notches 39 taking the form of recesses disposed on its longitudinal sides for fixing the position of the entraining member 13 or for fixing the position of a holder 16 inserted into the gap for the reception of prosthesis elements and the like, the diameter of the internal bore 8 being larger than the width of the gap 5, 5a measured perpendicularly to the longitudinal axis of the internal bore.

9. An anchoring member according to Claim 8, characterised in that after the introduction of the anchoring member 1 into the tissue, the entraining member 13 can be interchanged for a holder 16 of basically identical construction, the holder 16 being provided with a base plate 17 disposed on the projection 14 for the retention of prosthesis parts and the like.

10. An anchoring member according to Claim 9, characterised in that after the introduction of the holder 16 into the internal bore 8, the rear end of the anchoring member 1 can be closed by a stop screw 18, accompanied by the simultaneous fixation of the holder 16 in position.

11. An anchoring member according to Claims 9 and 10, characterised in that the holder 16 has a continuous bore 21 for the reception of a pin 22 whose front threaded portion cooperates with a matching thread 24 in the end of the internal bore 8.

12. An anchoring member according to Claim 11, characterised in that the pin 22 has an external thread 25 for the fixation of the holder 16 in position for cooperation with a matching internal thread 26 in the bore 21 disposed in the holder 16.

13. An anchoring member according to one of Claims 9-12, characterised in that a portion 28 of the holder 16 at least partially fills the bore 8, the top side of said portion 28 having at least in zones projections 29, 30, which for the fixation of the holder 16 in position cooperate with notches 31, 32 provided in the internal bore 8.

14. An anchoring member according to one of Claims 9-13, characterised in that the portion 28 of the holder 16 situated in the internal bore 8 only partially fills the latter and for the fixation of the holder 16 in position can be brought by means of wedge-shaped insert elements 36 by its projections 29, 30 into engagement with the appropriate notches.

15. An anchoring member according to one of Claims 9-14, characterised in that the portion of the holder 16 situated in the internal bore 8 has projections 38 for the fixation of the holder 16 in position via the agency of matching recesses 39 along the longitudinal side walls of the gap 5.

16. An anchoring member according to one of Claims 9-15, characterised in that the cover strip 6 comprises a flexible, relatively thin strip-shaped cover member 6 which can be gradually pressed into a free space left between the holder 16 and the internal bore 8 when the holder 16 is inserted in the gap 5.

17. An anchoring member according to one of the preceding claims, characterised in that the introduction end 3 of the anchoring member 1 tapers conically and has one or more slots 9 which are provided with cutting edges and which start from the open end portion of the anchoring member 1.

18. A device for anchoring a joint prosthesis and the like in bone tissue, comprising a mainly axially symmetrical anchoring member 1, made from a tissue-compatible material and provided for implantation in the tissue, and at least one holding means provided for the joint prosthesis, the anchoring member having an external thread 2 which starts from its rear end 4 and extends in the direction of the introduction end 3, characterised in that
- the anchoring member 1 has at least one slot-shaped gap 5, which is provided at a distance from the introduction end 3 in the surface formed with a thread and which extends mainly axially starting from the rear end of the anchoring member and is provided for the releasable reception of a cover member filling the gap while the anchoring member is being screwed into a bore previously prepared in the tissue; the anchoring member 1 is provided to be screwed into the bore previously prepared in the bone tissue close beside a joint and substantially parallel with the joint axis or the joint plane, and
- the anchoring member 1 is provided via the slot-shaped gap 5, 5a for the releasable reception of the holding means 16 provided for the joint prosthesis.

19. An anchoring device according to Claim 20, characterised in that it comprises two or more anchoring members 1.

## Revendications

1. Organe d'ancrage (1) se composant d'une matière bien tolérée par les tissus, prévu pour servir d'implant dans les tissus et conçu de manière essentiellement symétrique en rotation pour la fixation de prothèses, d'éléments d'articulation artificielle et similaires, l'organe d'ancrage présentant un filetage mâle (2) qui s'étend depuis sa partie extrême arrière (4) en direction de la partie extrême d'introduction (3), au moins un interstice en forme de fente (5, 5a) étant disposé à distance de la partie extrême d'introduction (3) dans la surface de l'enveloppe munie du filetage mâle (2), caractérisé en ce que l'interstice formant fente (5, 5a) s'étend de manière essentiellement axiale depuis la partie extrême arrière (4) de l'organe d'ancrage (1), l'organe d'ancrage étant prévu pour la réception amovible d'un organe de recouvrement remplissant l'interstice en forme de fente (5, 5a) pendant le vissage de l'organe d'ancrage (1) dans une cavité préparée à l'avance dans le tissu.

2. Organe d'ancrage selon la revendication 1, caractérisé en que ledit interstice (5, 5a) est muni, pendant la mise en place de l'organe d'ancrage (1) dans une cavité préparée à l'avance dans le tissu et pendant la période d'intégration osseuse, d'une baguette (6, 6a) amovible, protégeant l'interstice (5, 5a).

3. Organe d'ancrage selon la revendication 1 ou 2, caractérisé en ce que l'interstice (5, 5a) est prévu pour la réception et le positionnement fixe desdites parties de prothèses, articulations et parties d'articulation artificielle et similaires après le retrait de la baguette de recouvrement (6, 6a).

4. Organe d'ancrage selon l'une quelconque des revendications 1 à 3, caractérisé en ce que l'interstice (5, 5a) est une rainure fraisée dans l'organe d'ancrage dont les bords de délimitation sont prévus pour la fixation amovible de la baguette de recouvrement (6, 6a).

5. Organe d'ancrage selon l'une quelconque des revendications 1 à 4, caractérisé par un perçage interne (8), partant de la partie extrême arrière (4) de l'organe d'ancrage (1), ce perçage (8) étant relié audit interstice (5, 5a).

6. Organe d'ancrage selon la revendication 5, caractérisé en ce que la longueur du perçage interne (8) est un peu plus grande que la longueur de l'interstice en forme de fente (5, 5a) qui coopère avec ce dernier.

7. Organe d'ancrage selon la revendication 5 ou 6, caractérisé en ce que la baguette de recouvrement (6, 6a) se compose d'un toc d'entraînement (13) qui est prévu pour effectuer l'insertion et le recouvrement de ladite fente (5, 5a) en forme d'interstice au moyen d'au moins une saillie axiale (14) en remplissant au moins partiellement le perçage interne (8) et qui est conçu de manière à faire tourner l'organe d'ancrage (1) lors de la rotation du toc d'entraînement (13).

8. Organe d'ancrage selon la revendication 7, caractérisé en ce que l'interstice en forme de fente (5), comprenant des encoches (39) disposées sur ses bords longitudinaux et formant des cavités, assure le positionnement fixe du toc d'entraînement (13) ou le positionnement fixe d'un support (16) inséré dans l'interstice pour le logement d'éléments de prothèse et similaire, le diamètre du perçage interne (8) étant supérieur à la largeur de l'interstice (5, 5a), qui est mesurée perpendiculairement à l'axe longitudinal du perçage interne (8).

9. Organe d'ancrage selon la revendication 8, caractérisé en ce qu'après avoir inséré l'organe d'ancrage (1) dans le tissu, le toc d'entraînement (13) peut être échangé contre un support (16) de configuration sensiblement similaire, le support (16) étant muni d'une plaque de base (17) disposée sur la saillie (14) dans le but de maintenir des éléments de prothèse et similaires.

10. Organe d'ancrage selon la revendication 9, caractérisé en ce que la partie extrême arrière de l'organe d'ancrage (1) peut être fermée par une vis d'obturation (18) après l'insertion du support (16) dans le perçage interne (8) tout en fixant la position du support (16).

11. Organe d'ancrage selon la revendication 9 ou 10, caractérisé en ce que le support (16) présente un perçage continu (21) pour le logement d'un boulon (22) dont la section de filet avant coopère avec un filetage correspondant (24) au fond du perçage interne (8).

12. Organe d'ancrage selon la revendication 11, caractérisé en ce que le boulon (22) présente un filetage mâle (25) dans le but de fixer en position le support (16) pour coopérer avec un filetage femelle (26) correspondant dans le perçage (21) disposé dans le support (16).

13. Organe d'ancrage selon l'une quelconque des revendications 9 à 12, caractérisé en ce que le support (16) remplit le perçage (8) du moins partiellement par une partie (28), la partie (28) présentant sur sa face supérieure des protubérances (29, 30) réparties au moins par zone, lesquelles protubérances coopèrent avec les encoches (31, 32) se trouvant dans le perçage interne (8) en vue de fixer le support (16) en position.

14. Organe d'ancrage selon l'une quelconque des revendications 9 à 13, caractérisé en ce que la partie (28) du support (16), se trouvant dans le perçage interne (8), ne remplit ledit perçage que partiellement et peut s'engager par ses protubérances (29, 30) dans les encoches correspondantes au moyen d'éléments d'insertion cunéiformes (36) afin de fixer le support (16) en position.

15. Organe d'ancrage selon l'une quelconque des revendications 9 à 14, caractérisé en ce que la partie du support (16) se trouvant dans le perçage interne (8) présente des protubérances (38) afin de réaliser un positionnement fixe du support (16) avec des cavités correspondantes (39) le long des parois latérales longitudinales de l'interstice (5).

16. Organe d'ancrage selon l'une quelconque des revendications 9 à 15, caractérisé en ce que la baguette de recouvrement (6) se compose d'une baguette de recouvrement (6) flexible relativement mince en forme de bandelette qui peut être enfoncée progressivement dans un espace libre entre le support (16) et le perçage interne (8) au moment de l'introduction du support (16) dans l'interstice (5).

17. Organe d'ancrage selon l'une des revendications précédentes, caractérisé en ce que la partie extrême d'introduction (3) de l'organe d'ancrage (1) se termine en pointe conique et présente une ou plusieurs fentes (9) qui sont munies d'arêtes vives et qui s'étendent depuis la section ouverte de fond de l'organe d'ancrage (1).

18. Dispositif d'ancrage d'une prothèse d'articulation ou équivalent dans le tissu osseux se composant au moins d'un organe d'ancrage (1) réalisé en une matière bien tolérée par les tissus, prévu pour servir d'implant dans les tissus et conçu de manière essentiellement symétrique, et d'au moins un support (16) prévu pour la prothèse d'articulation, l'organe d'ancrage présentant un filetage mâle (2) qui s'étend depuis sa partie extrême arrière (4) en direction de la partie extrême d'introduction (3), caractérisé en ce que
- l'organe d'ancrage (1) comprend au moins un interstice en forme de fente (5, 5a) disposé à distance de la partie extrême d'introduction (3) dans la surface de l'enveloppe munie du filetage mâle, interstice qui s'étend de manière essentiellement axiale depuis l'extrémité arrière de l'organe d'ancrage, et en ce que l'organe d'ancrage (1) est prévu pour la réception amovible d'un organe de recouvrement remplissant l'interstice en forme de fente pendant le vissage de l'organe d'ancrage (1) dans une cavité préparée à l'avance dans le tissu, et en ce que l'organe d'ancrage (1) est prévu pour le vissage dans le perçage préparé à l'avance tout près d'une articulation et sensiblement parallèle à l'axe d'articulation ou au plan d'articulation dans le tissu osseux, et
- en ce que l'organe d'ancrage (1) est prévu pour le logement amovible de la fixation (16) prévue pour la prothèse d'articulation par l'intermédiaire de l'interstice en forme de fente (5, 5a).

19. Dispositif d'ancrage selon la revendication 20, caractérisé en ce qu'il comprend deux ou plusieurs organes d'ancrage (1).
